# DEMANDE DE BREVET EUROPEEN

(11) **EP 0 888 769 A1**
(43) Date de publication de la demande: **07.01.1999**
(21) Numéro de dépôt: 98401437.3
(22) Date de dépôt: 12.06.1998
(51) Int. Cl.: A61K 7/48, A61K 47/36

(54) **Compositions cosmétiques à base de lyophilisats d'alginates alcalins**

(30) Priorité: 13.06.1997 FR 9707336
(71) Demandeur: Dogliani, Alain, 83380 Les Issambres (FR)
(72) Inventeur: Dogliani, Alain, 83380 Les Issambres (FR)
(74) Mandataire: Petit, Hélène

(57) **Abrégé**

- Nouvelles compositions cosmétiques constituées de lyophylisats composés d'alginates alcalins hydrosolubles et de composés actifs hydratants, nourrissants, restructurants ou raffermissants et masques de beauté en résultant.
- Nouveaux vecteurs de pénétration de composés actifs lyophilisés dans la peau constitués par des lyophilisats d'alginates alcalins hydrosolubles.

## Description

La présente invention a pour objet de nouvelles compositions cosmétiques caractérisées en ce qu'elles sont constituées de lyophilisats composés d'alginates alcalins hydrosolubles et de composés actifs hydratants, nourrissants, restructurants ou raffermissants.

Les lyophilisats selon l'invention se présentent sous forme de feuilles plus ou moins épaisses, rigides et peuvent être appliquées directement sur la peau du visage ou du corps. Elles sont alors réhydratées à l'aide d'eau pure, d'eau marine, ou de toute autre lotion compatible. Elles se transforment en un gel que l'on peut répartir facilement sur toute le surface de peau pour former un masque de quelques centimètres d'épaisseur. Environ 10 minutes après l'application, le gel a totalement pénétré dans la peau, sans laisser aucun résidu.

Les lyophilisats selon l'invention présentent d'une part des propriétés désensibilisantes et d'autre part des propriétés exceptionnelles de pénétration qui ont été mises en évidence par des analyses systématiques, notamment à l'aide de cellules de diffusion de type Frantz.

Il apparait que les alginates alcalins lyqphilisés associés aux composés actifs cosmétiques également lyophilisés jouent à l'égard de ces derniers le rôle de vecteurs, leur permettant une pénétration remarquable.

La présente invention a également pour objet de nouveaux vecteurs de pénétration de la peau constitués pour des lyophylisats d'alginates alcalins hydrosolubles.

Les alginates alcalins lyophilisés jouent également un rôle d'actif cosmétique hydratant, de désensibilisant et de stabilisant.

Le procédé de lyophilisation est une technique connue, notamment pour conserver des protéines, qui consiste à congeler la préparation à lyophiliser, puis à éliminer l'air qu'elle contient en le plaçant dans une chambre munie d'une pompe à vide, à sublimer la glace sous vide et éliminer la vapeur d'eau.

Les nouvelles compositions cosmétiques selon l'invention comportent avantageusement 70 à 90 % de principes actifs et 10 à 30 % d'alginates.

Les alginates utilisés dans le cadre de la présente invention sont des alginates alcalins hydrosolubles, tels que l'alginate de sodium, l'alginate de potassium, ou des mélanges de ceux-ci.

Les composés actifs cosmétiques sont des composés hydratants, nourrissants, restructurants, raffermissants et tenseurs, additionnés éventuellement de composés complémentaires tels que des extraits de plantes, des algues micro-éclatées, des minéraux et des oligo-éléments, des protéines marines.

La présente invention a notamment pour objet des préparations cosmétiques hydratantes constituées de lyophilisats d'alginates et de collagène marin. Les masques au collagène marin lyophilisé selon l'invention présentent des propriétés hydratantes très supérieures à celles des feuilles de collagène bovin lyophilisé. Le gel qui constitue le masque est directement assimilable par la peau et pénètre complètement de telle sorte que la peau apparait nette et douce.

Ceci est un avantage considérable par rapport aux feuilles de collagène bovin utilisées jusqu'à présent, dont le substrat devait être retiré après la pose.

Les analyses de libération des composés actifs des masques obtenus avec les lyophilisats de l'invention montrent que l'on retrouve au minimum 95 % des composés actifs dans la peau après une pose moyenne de 10 minutes.

La présente invention permet ainsi d'obtenir une large gamme de masques très actifs qui peuvent être employés pour tous types de peaux, même les plus sensibles.

On donne ci-après, à titre d'illustration, quelques exemples non limitatifs de produits obtenus avec les compositions de l'invention.

### Exemple N° 1. Masque facial hydratant :

Alginate de sodium
Collagène natif marin
Huile essentielle de sauge officinale

Dans cet exemple, l'alginate représente 12 % du poids total de la composition, le collagène natif marin, principe actif hydratant, 87 % et l'huile essentielle de sauge officinale 1 %.

### Exemple N° 2. Masque facial tenseur :

Alginate de sodium
Elastine marine
Huile essentielle de romarin

Dans cet exemple, l'alginate représente 12 % du poids total de la composition, l'élastine marine, principe actif hydratant et tenseur, 87 % et l'huile essentielle de romarin 1 %.

### Exemple N° 3. Masque facial régénérant :

Alginate de sodium
Nucléotides marins
Huile essentielle de myrte

Dans cet exemple, l'alginate représente 12 % du poids total de la composition, les nucléotides marins, principe actif régénérant, 87 % et l'huile essentielle de myrte 1 %.

### Exemple N° 4. Masque facial raffermissant :

Alginate de sodium
Collagène natif marin
Algues micro-éclatées
Extrait de prêle
Huile essentielle de lavandin

Dans cet exemple, l'alginate représente 12 % du poids total de la composition, le collagène marin, principe actif hydratant, 30 % et les autres constituants raffermissants 58 %.

Dans tous ces exemples, après 10 à 15 minutes d'application, la préparation a pénétré complètement dans la peau sans laisser aucun résidu.

Les masques obtenus à partir des lyophilisats de la présente invention sont exceptionnellement actifs puisque leurs constituants sont absorbés à un minimum de 95 % par la peau.

Ces masques présentent également une biocompatibilité et une tolérance remarquables qui se manifestent par l'absence totale de toute irritation cutanée ou allergique telles que rougeurs, échauffements, vasodilatations, desquamations ou démangeaisons.

Cet avantage est particulièrement sensible pour les produits qui doivent rester au contact de la peau pendant une durée prolongée.

## Revendications

1. Compositions cosmétiques constituées de lyophilisats composés d'alginates alcalins hydrosolubles et de composés actifs d'origine marine.

2. Compositions selon la revendication 1, caractérisées en ce que le composé actif est du collagène marin, de l'élastine marine, des nucléotides marins ou un de leurs mélanges.

3. Compositions selon la revendication 2, caractérisées en ce que le composé actif cosmétique est du collagène natif marin.

4. Compositions selon les revendications 1 à 3, caractérisées en ce qu'elles contiennent de 10 à 30 % en poids d'alginates et de 70 à 90 % en poids de composés actifs cosmétiques.

5. Compositions selon les revendications 1 à 4, caractérisées en ce que les alginates sont des alginates alcalins hydrosolubles choisis dans le groupe comprenant l'alginate de sodium, l'alginate de potassium ou un mélange de ceux-ci.

6. Masques cosmétiques réalisés en hydratant les lyophilisats selon les revendications 1 à 5.

7. Vecteurs de pénétration de composés actifs lyophilisés dans la peau, caractérisés en ce qu'ils sont constitués par des lyophilisats d'alginates alcalins hydrosolubles, selon les revendications 1 à 5.
